# EUROPEAN PATENT SPECIFICATION

(11) **EP 1 651 201 B1**
(45) Date of publication and mention of the grant of the patent: **02.01.2008**
(21) Application number: 04761468.0
(22) Date of filing: 23.07.2004
(51) Int. Cl.: A61K 31/122, A61K 49/06, A61P 43/00

(54) **NECROSIS AVID TRACER AGENT**
NEKROSE-AVIDES TRACER-AGENT
AGENT TRACEUR PRESENTANT UNE AVIDITE POUR LA NECROSE

(30) Priority: 25.07.2003 GB 0317467
(43) Date of publication of application: 03.05.2006
(73) Proprietor: K.U.Leuven Research & Development, 3000 Leuven (BE)
(72) Inventor: NI, Yicheng, B-3020 Herent (BE); BORMANS, Guy, B-3110 Rotselaar (BE); MARCHAL, Guy, B-3052 Blanden (BE); VERBRUGGEN, Alfons, B-3012 Wilsele (BE)
(74) Representative: Bird, Ariane
(86) International application number: PCT/BE2004/000107
(87) International publication number: WO 2005/009423

(56) References cited:
- HUYGHE, D. ET AL.: "Preparation of mono(123J)-iodohypericin, biodistribution in a mouse RIF-1 tumour model and protein binding in himan plasma" JOURNAL OF NUCLEAR MEDICINE, vol. 44, no. 5 Suppl., May 2003 (2003-05), page 303P, XP009038229

## Description

### FIELD OF THE INVENTION

The present invention concerns to the use of phenanthro[1,10,9,8-opqra]perylene-7,14-dione derivatives, and more specifically hypericin or its derivatives, as necrosis or infarct specific agents. The phenanthro[1,10,9,8-opqra]perylene-7,14-dione derivatives can be labeled with a radionuclide, a radiopaque material or a material enhancing the effects magnetic resonance imaging.

### BACKGROUND OF THE INVENTION

Non-invasive "hot spot imaging" and localization of necrotic tissue may be helpful for the diagnosis of different disorders. This is especially true in the case of ischemic myocardial injury, where imaging using necrosis avid agents could help in measuring infarct size. This could allow the rapid use of interventions for myocardial salvage through early identification of the acute event, in this way improving the clinical outcome. Also other cardiovascular disorders associated with cardiac cell death could be visualized and diagnosed, such as cardiomyopathies, myocarditis and heart transplant rejection (*1*).
In Nuclear Medicine, only a limited number of "hot spot imaging" agents have been used or proposed. ^{99m}Tc-pyrophosphate was the first of them and is supposed to bind to necrotic myocardial tissue by targeting calcium phosphate deposited in the mitochondria of infarcted or severely injured myocardial tissue (*2*). However, ^{99m}Tc-pyrophosphate scintigraphy has never gained widespread use because of its limited diagnostic accuracy and relatively poor stability that may lead to the presence of pertechnetate and so result in imaging of the cardiac chambers. More recently, ¹¹¹In-labeled murine monoclonal antimyosin Fab antibody fragments were introduced for infarct-avid scintigraphy (*3,4*). This so-called ¹¹¹In-antimyosin has a selective affinity for the intracellular heavy chain of cardiac myosin, which is exposed when the integrity of the sarcolemma is lost as a result of cell damage (*5,6*). It is incorporated in the necrotic myocardium in an inverse relationship to regional flow, with maximum uptake in areas with severe flow impairment, although its uptake is more intense in myocardial infarcts with reperfusion than in those with persistent coronary occlusion (*7*). It was also found useful for detection of acute or chronic diffuse myocardial damage in allograft rejection in cardiac transplantation (*8,9*), doxorubicin-induced cardiotoxicity (*10*), acute myocarditis (*11*,*12*) and various cardiomyopathies (*13*,*14*). Although ¹¹¹In-antimyosin was recently approved by the FDA for clinical use, the limitation of approved indications to ischemic heart disease has resulted in cessation of commercial production.

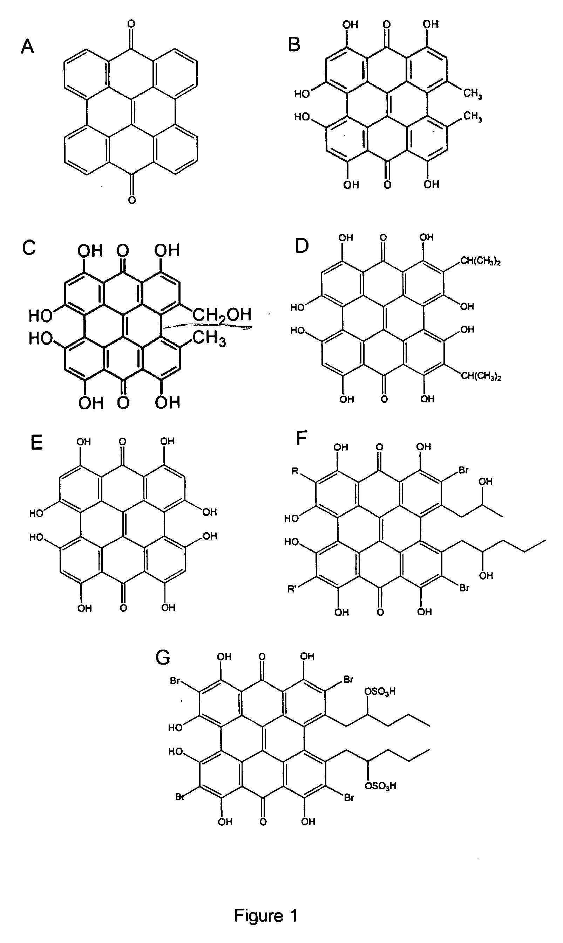

A few years ago, it was observed that the complex of 99mTc with glucaric acid, a simple dicarboxylic acid sugar, localized in canine reperfused myocardial infarction soon after injection (15,16). The tracer agent is supposed to bind to positively charged histones within disintegrated nuclei and reduced subcellular organelle proteins in necrotic myocytes (17). It has the advantage of a rapid blood pool clearance, a good target to background ratio, lack of toxicity and antigenicity and is claimed to have a good sensitivity and specificity for detection of early irreversible myocyte injury. On the other hand, its uptake is limited to the first 9 hours after the onset of acute myocardial infarction because of the relatively rapid disintegration of the positively charged histones. Further clinical trials have to reveal its real clinical usefulness.
In the field of Magnetic Resonance Imaging (MRI), it has recently been reported that some porphyrin derivatives that were originally developed as tumor-seeking contrast agents (18) have avidity only for nonviable tissues (typically necrosis) instead of viable tumoral cells and could therefore be re-categorized as necrosis-avid contrast agents (NACAs) (19-21). The targetability of these agents for necrosis has elicited novel applications for MRI visualization of acute myocardial infarction (22-33) and therapeutic tissue ablation (34).
The present invention relates to phenanthro[1,10,9,8-opqra]perylene-7,14-dione (Fig. 1A) derivatives. Typical examples of such derivatives are the natural photosensitizing and photosensory pigments like hypericin (Fig. 1B), pseudohypericin (Fig. 1 C), stentorin (Fig. 1D), the fringelites (Fig. 1E), the gymnochromes (Gymnochrome B (Fig. 1F), Gymnochrome D (Fig. 1G). Isogymnochrome D) and blepharismin (P.S. Song, 1995, J Photoscience 2, 21-35). Huyghe et al in Journal of Nuclear Medicine (2003) 44(5) 303P teaches the preparation of mono(123-I)-iodohypericin, biodistribution in a mouse tumor model and protein binding in human plasma.

### SUMMARY OF THE INVENTION

The present invention is based on the unexpected finding that the use of a radiolabeled phenanthro[1,10,9,8-opqra]perylene-7,14-dione derivative, more particularly mono-[123]iodohypericin, allowed the in situ visualistion of infarcted, ischemic or necrotic tissue. Therefore, the main object of the present invention relates to the use of a compound comprising phenanthro[1,10,9,8-opqra]perylene-7,14-dione for the manufacture of an imaging agent for obtaining an image of ischemic, infarcted or necrotic tissue. In another aspect this invention provides a pharmaceutical composition comprising a hypericin or a derivative thereof being conjugated to a radiopaque material or a radionucleide, wherein said radionucleide is selected from the group consisting of Tc-99m, I-125, I-111, In-113m and G-67. In another aspect this invention provides a pharmaceutical composition comprising a phenanthro[1,10,9,8-opqra]perylene-7,14-dione compound conjugated to a radionucleide or a radiopaque material wherein said phenanthro[1,10,9,8-opqra]perylene-7,14-dione compound is selected from the group consisting of pseudohypericin, stentorin, the fringelites, the gymnochromes, and blepharismin.

### DETAILED DESCRIPTION OF THE INVENTION

### List of Figures

Figure 1: Structure of phenanthro[1,10,9,8-opqra]perylene-7,14-dione and of different natural occuring pigments derived thereof: A: phenanthro[1,10,9,8-opqra]perylene-7,14-dione, B: hypericin, C: pseudohypericin, D: stentorin, E: the fringelite D, F: Gymnochrome B, G: Gymnochrome D.
Figure 2: Chemical structure of hypericin (R = H) and mono-[123I]iodohypericin (MIH) (R = 1231).
Figure 3: Ex vivo images of rat liver obtained 30 min p.i. of MIH.
   intact liver: (1) autoradiography of liver slice; (2) TTC staining of adjacent tissue block; (3) H&E staining of the same liver slice.
   necrotic liver: (1) autoradiography of liver slice; (2) TTC staining of adjacent tissue block; (3) H&E staining of the same liver slice.
Figure 4: Ex vivo images of rat liver obtained 24 h p.i. of MIH
   intact liver: (1) autoradiography of liver slice; (2) TTC staining of adjacent tissue block; (3) H&E staining of the same liver slice.
   necrotic liver: (1) autoradiography of liver slice; (2) TTC staining of adjacent tissue block; (3) H&E staining of the same liver slice.
Figure 5: SPECT images (coronal section) obtained at 24 h (left) and 48h (right) post injection of MIH in a rabbit with myocardial infarction. The radioactivity can be persistently found in the heart region and to a lesser extent in the thyroid and gut.
Figure 6: Ex vivo images of rabbit heart. After TTC staining of a 5-mm thick slice (A), a 50-µm frozen slice (B) and corresponding autoradiographic image (C) were obtained and matched when overlapped (D).

### DESCRIPTION

The present invention is based on the surprising finding that hypericin, has a preferential distribution and retention in infarcted, ischemic or necrotic tissue following the adminstration thereof to a human or animal subject. Hypericin is a naturally occuring photosensitizer, which belongs to the group of pigments derived from the fundamental chromophore phenanthro[1,10,9,8-opqra]perylene-7,14-dione. Next to synthetic derivatives of hypericin, this group further comprises other naturally occuring pigments such as stentorin, the fringelites, the gymnochromes, and blepharismin. Therefore, a first object of the present invention is the use of compounds derived from the phenanthro[1,10,9,8-opqra]perylene-7,14-dione as for the manufacture of an imaging agent for obtaining an image of ischemic, infarcted or necrotic tissue. In a preferred embodiment said compounds have photosensitizing properties. In a more preferred embodiment said compounds are selected out of the group of the naturally occurring pigments consisting of hypericin, pseudohypericin, stentorin, the fringelites, the gymnochromes, and blepharismin and derivatives thereof. In a most preferred embodiment the invention relates to the use of hypericin or a derivative thereof as necrosis or infarct specific agent.
It is an aspect of the invention to use the said phenanthro[1,10,9,8-opqra]perylene-7,14-dione derived compounds as necrosis-avid contrast agents as imaging agents in nuclear medicine imaging. In a more preferred embodiment, a radiolabeled derivative of hypericin is used. For imaging purposes any of the well-known medical radionuclides can be used as radiolabel. Suitable radionuclides include Tc-99m, I-123, I-125, I-111, In-113m, G-67, or other suitable gamma-emitters. Preferably radio-labeled hypericin is used, more preferably hypericin is labeled on carbon 2 atom, in ortho-position of the phenolic group with the most acidic characteristics.
The person skilled in the art is aware that imaging techniques other than nuclear imaging are used for the purpose of visualizing ischemic or infarcted tissue, such as X-ray and magnetic resonance. The use of a contrast agent in an X-ray procedure requires that the agent is labeled with a radiopaque material. Suitable radiopaque materials are well known and include iodine compounds, barium compounds, gallium compounds, thallium compounds, and the like. Specific examples of radiopaque materials include barioum diatrizoate, ethiodized oil, gallium citrate, iocarmic acid, iocetamic acid, iodamide, iodipamide, iodoxamic acid, iogulamide, iohexol, iopamidol, iopanoic acid, ioprocemic acid, iosefamic acid, ioseric acid, iosulamide meglumine, iosumetic acid, iotasul, iotetric acid, iothalamic acid, iotroxic acid, ioxaglic acid, ioxotrizoic acid, ipodate, meglumine, metrizamide, metrizoate, propyliodone and thallous chloride. Labels that can be detected or that enhance the effects of magnetic resonance imaging include gadolinium, copper, iron and chromium. It is preferred that these metal atoms be prepared in the form of a conventional organometallic chelates, which are then bound to the contrast agent.
In a second object, the present invention provides a method for the imaging of ischemic or infarcted tissue. Said method comprises the parenteral administration of an effective amount of an appropriately labeled contrast agent of the present invention to an animal or human subject. The dosage may vary depending upon the imaging technique used, the area and tissue to be imagined, the age and condition of the subject and other factors, which a skilled practitioner would consider. After injection, sufficient time is allowed for the said imaging agent to accumulate at the site of the diseased tissue. In a preferred embodiment the method of the present invention, radiolabeled hypericin is injected parenterally, preferably intravenously, into a subject. After allowing sufficient time the subject is scanned with a gamma camera.

### Illustrative embodiment

### Materials en Methods

### Synthesis and preparation of mono-[¹²³I]iodohypericin

MIH was synthesized in no-carrier added form using a standard electrophilic radioiodination method in the presence of peracetic acid and was purified with high pressure liquid chromatography (HPLC) as described previously (37). After purification the MIH solution was evaporated to dryness at 60 °C under a flow of nitrogen and the tracer agent was redissolved in water/polyethylene glycol (PEG) 400 (8:2, V/V) immediately before injection.

### Animal models of Necrosis

All animal experiments were conducted with the approval of the institutional ethical committee.
**1. Reperfused hepatic infarction:** six adult Wistar rats weighing 400-450 g were anesthetized with intraperitoneal injection of pentobarbital (Nembutal^{®}, Sanofi Santé Animale, Brussels, Belgium) at a dose of 40 mg/kg. Under laparotomy, reperfused hepatic infarction was induced by temporarily clamping the hilum of the right liver lobes for 3 hours. After reperfusion by declamping hepatic inflow, the abdominal cavity was closed with 2-layer sutures and the rats were allowed to recover for 8h after the surgery (19,37).
2. **Occlusive myocardial infarction**: A New-Zealand adult rabbit weighing about 5 kg was sedated with intramuscular injection of a mixed solution of Ketalar (ketamine hydrochloride, Pfizer, Brussels, Belgium) at 15 mg/kg and Rompun (xylazine hydrochloride, Bayer, Leverkusen, Germany) at 2.5 mg/kg. The animal was then anaesthetized with an intravenous bolus injection of Nembutal at 15 mg/kg followed by IV infusion of Nembutal at 0.1 mg/kg/min through an ear vein. Under a cold-light laryngoscope with No. 1 pediatric blade (Riester®, Jungingen, Germany), the animal was intubated with a 3.5-mm endotracheal tube, which was then connected to an artificial ventilator (Mark 7 respirator, Bird Corporation, Palm Springs, CA). An open chest surgery of left posterolateral thoracotomy was performed. The pericardium was opened to expose the left circumflex coronary artery (LCX). A loose snare loop of a 3-0 silk suture was applied around the artery and tightened with ligature. The chest was closed after evacuation of the pneumothorax.

### Tracer administration

Five hours after reperfusion of the hepatic infarction, rats were intravenously injected with 3.7 MBq of MIH through a tail vein.

Two hours after reperfusion of the myocardial infarction, the rabbit was intravenously injected with 130 MBq MIH via an ear vein.

### SPECT imaging:

The rabbit imaging measurements were performed using a two-detector gamma camera (E-cam, Siemens Medical Systems, Erlangen, Germany). The rabbit was secured to the head-holder of the patient bed with the organ of interest in the center of the field of view. SPECT projections were collected using the following acquisition parameters: 120 projections over 360° per detector head (step & shoot mode), 25 s per projection, a matrix size of 256x256, a pixel size of 2.4 mm, and a non-circular orbit. The images were reconstructed with maximum-likelihood expectation-maximization, applying 47 iterations.

*In vivo* SPECT was conducted under the same anesthetic regimes as that for surgery of the rabbit. The rabbit was imaged at 24 and 48 hours after injection of MIH.

### Animal Sacrifice, Tissue Sampling and TTC staining

Thirty minutes (n=3) or 48 hours (n=3) post-injection, the rats were sacrificed with an intravenous overdose of Nembutal. The rats were dissected and the organs were weighed and counted in a gamma counter (Wallac Wizard, Turku, Finland). Normal (dark red) and necrotic (pale) parts of the liver were prelevated for either tissue counting or autoradiography and tissue staining For the latter two techniques, tissues were quickly frozen in isopentane at -50°C.

For the rabbit experiment, the heart was cut perpendicularly to the long axis into 5-mm thick slices starting from the apex. The slices were immersed in a buffered triphenyltetrazolium chloride (TTC) solution for staining during 10 min at 37°C. From one slice, samples of normal and infarcted myocardium (guided by TTC staining) were weighed and counted in a gamma counter. Another slice was quickly frozen in isopentane at -50°C.

The liver tissue chunk remaining after obtaining frozen sections for autoradiography, was immersed in a TTC solution for staining during 10 min at 37°C. The tissue chunk was then placed on a transparant foil with the sectioned surface facing down and scanned with an optical scanner (Hewlett Packard ScanJet 5400C). After this staining, normal liver and myocardium tissues appeared brick red and the necrotic areas were unstained (23,25,28-33).

### Ex vivo autoradiography and H&E staining

The frozen liver and heart tissues were cut at -20°C into 20-50 µm serial sections and exposed overnight to a high performance storage phosphor screen (Super resolution screen, Canberra-Packard, Ontario, Canada). The screen was read using a Phosphor Imager scanner (Cyclone^{™}, Canberra-Packard). The images were analyzed using Optiquant^{™} software (Canberra-Packard). Afterwards, these same sections were stained with haematoxylin-eosin (H&E) using the conventional procedure. These H&E stained sections were digitally scanned and the obtained images were corrected for brightness and contrast.

### Tissue Radioactivity Counting

Guided by TTC staining, the liver lobes of the rat and heart sections of the rabbit were sampled for normal and necrotic parts, weighed separately and counted for radioactivity using a 3-in. Nal(TI) scintillation detector mounted in a sample changer (Wallac Wizard, Turku, Finland). Corrections were made for background radiation and physical decay during counting. The activity in the normal and infarcted parts of the liver and heart was then expressed as counts per gram tissue.

### Results

### Liver lobe infarction in rats

*Tissue radioactivity counting.* At 30 minutes p.i. the ¹²³I concentration is high in the blood, the spleen, the liver and the lungs. The concentration in the intact liver lobe is 2 times higher then in the necrotic liver lobe. Due to the clearance to the bile, the concentration of ¹²³I at 24 h p.i. increases in the small and large intestine and decreases in the intact liver whereas it remains almost unchanged for the necrotic region. The thyroïd concentration is quite high indicating deiodination. (Table 1)

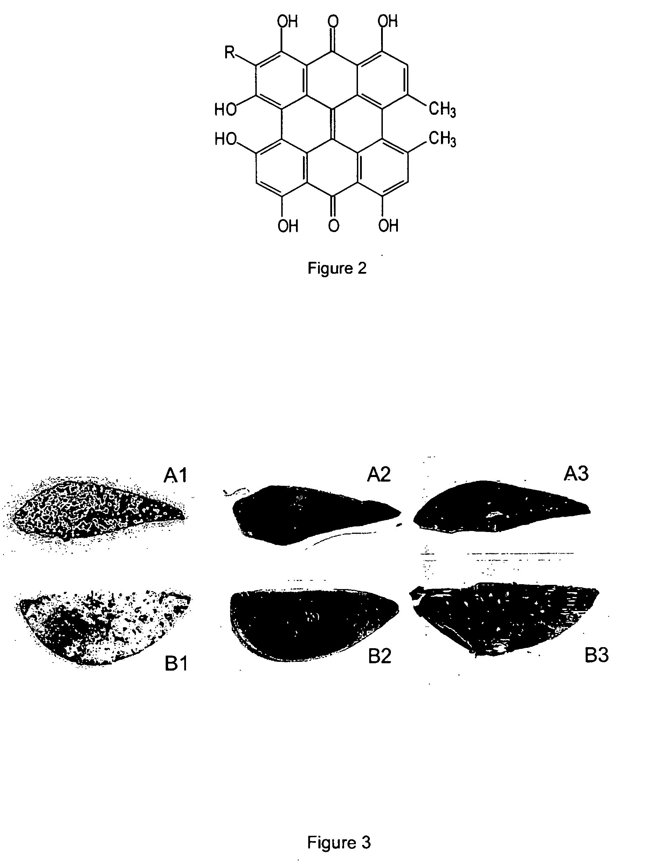

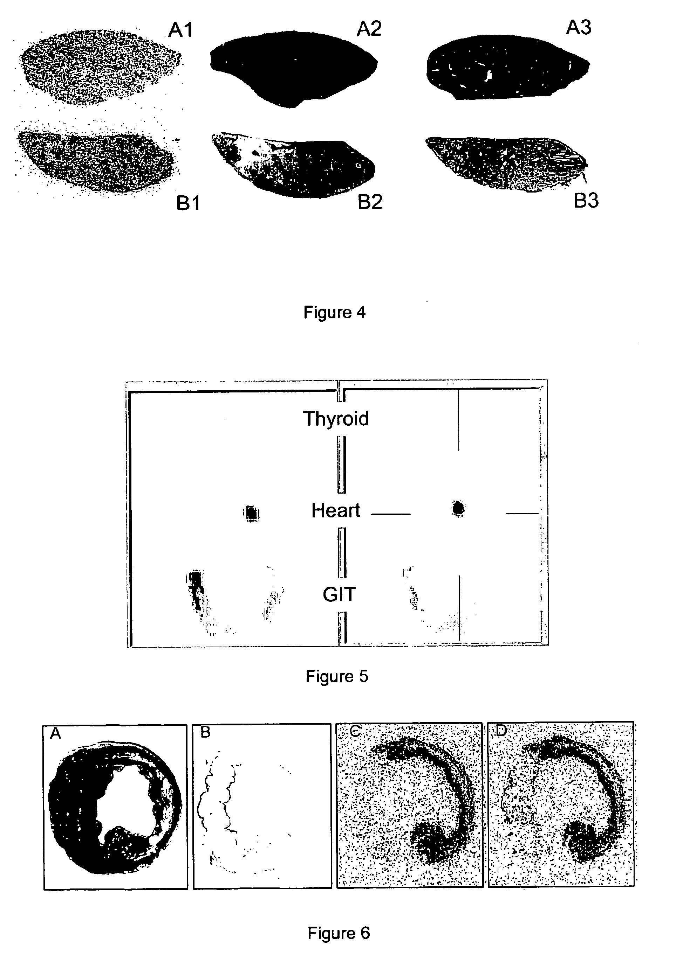

*Ex vivo autoradiography and histochemical Staining. Figure 3 and 4* compare the outcomes of autoradiography, H&E and TTC staining at respectively 30 min and 24 h p.i. of MIH. The slices of intact liver obtained at 30 min p.i. (Fig. 3) and 24 h p.i. (Fig. 4) both show a homogeneous deep red TTC staining (A2) and purple H&E staining (A3) confirming the viability of the tissue. Pronounced radioactivity uptake with a dotted distribution at 30 min p.i. was observed on the autoradiography of the slice of the intact liver (Fig 3, A1). At 24 h p.i. activity from the intact liver has cleared (Fig 4, A1).

The slices obtained from the infarcted liver lobe at 30 min p.i. (Fig. 3) show a low uptake of MIH (B1) in regions which appear light red on TTC staining (B2) and pink after H&E staining indicating necrosis (B3). In some regions a high uptake of MIH with similar distribution as in the intact liver slice is observed. This region corresponds to regions stained deep red with TTC and purple with H&E, indicating that these regions are viable. It should be noted that the low contrast between viable and necrotic tissue after TTC staining is due to the early timepoint after induction of infarction and that the shape of the TTC stained specimens deviates from the autoradiograms and H&E slices as the former is obtained after thawing the tissue from which the slices of the latter are obtained in frozen conditions.

The slices obtained from the infarcted liver tissue at 24 h p.i. (Fig. 4) show a relatively high uptake of MIH (B1) in regions which are stained pink by TTC (B2) and pink-purple (B3) by H&E confirming necrosis. In regions which are stained deep red (viable tissue) or pale (no reperfusion) by TTC, corresponding to respectively pink or purple regions after H&E staining, absence of radioactivity is observed on the corresponding autoradiogram

### Myocardial infarction in the rabbit

*In vivo SPECT imaging:* Twenty four and 48 hours post injection of MIH in the rabbit with occlusive myocardial infarction, the coronal section view after iterative reconstruction of whole body SPECT persistently displayed only one hot spot in the anatomical area of the heart. To a lesser extent, uptake of activity could also be seen in the regions of thyroid and intestines (Fig. 5).

*Tissue radioactivity counting:* TTC staining enabled selective sampling of reddish normal myocardium and whitish infarcted myocardium for radioactivity quantification. The results showed a 7-fold higher radiotracer concentration in the infarcted tissue than in the healthy tissue.

*Ex vivo histochemical staining and autoradiography:* Figure 6 compares TTC staining of a 5 mm thick slice, a 50-µm frozen slice with the corresponding autoradiographic image and a superposition of the two images. On the TTC stained specimen with right ventricular wall attached (Fig. 6a), the transmural infarction involves the entire anterior and lateral wall of the left ventricle including the posterior papillary muscle (negative staining with TTC). On the frozen slice with right ventricular wall removed (Fig. 6b), the normal myocardium at the posterior wall and interventricular septum are stained only superficially with TTC (by the nature of this macroscopic staining technique). On autoradiography (Fig. 6c,d), a "doughnut" pattern of radioactive uptake appears only in the infarcted region. However, the highest activity (in red) is found mainly subendocardially and near the lateral border zone, a sign characteristic for occlusive myocardial infarct (*39,40*).

### Discussion

As hypericin is a polyphenolic polycyclic quinone, it can be labelled efficiently and quite simply with radioiodine by electrophilic substitution in ortho position of a phenol (37). Structural analysis of the radioiodinated derivative has shown that in this way one iodine-123 radionuclide is reproducibly introduced on carbon atom 2, in ortho position of the phenolic group with the most acidic characteristics (Fig. 2). The resulting mono-[¹²³I]iodohypericin (MIH) could efficiently be separated from the starting material hypericin by reversed phase HPLC and was obtained with an over 99% purity in non-carrier added form.

HPLC purified MIH was injected in rats with partial reperfused liver infarction.

Thirty minutes after intravenous injection of MIH in rats, still a high concentration of ¹²³I is present in the blood and MIH shows a pronounced uptake in the liver, the spleen and the lungs (Table 1). At 30 min p.i. the concentration in intact liver is twice as high as the concentration in the necrotic liver lobe. MIH is a lipophilic compound which is cleared from plasma by hepatobiliary excretion (*37, 41*) and this results in a high concentration in normal liver at 30 minutes and a secretion of the activity via the bile to the gastrointestinal tract. This results in a lower concentration in the blood, the lungs, the spleen and the intact liver and an increased concentration in the Gl tract at 24 h post injection. The high concentration in the thyroid indicates that in vivo deiodination of MIH occurs. In contrast to the intact liver, the concentration of ¹²³I in the necrotic liver remains almost constant between 30 minutes and 24 hours post injection and at the latter time point the concentration in the necrotic liver tissue is 3.2 times higher than in the intact liver tissue. Since it can be assumed that the hepatocyte transporter systems of the necrotic liver lobe will not be functional anymore, the mechanism of retention of MIH in the necrotic liver lobe will be different from that in the intact liver tissue. At 24 hours p.i., the concentration ratios of necrotic liver vs. blood and necrotic liver vs. muscle are very high (respectively 17 and 57).

Autoradiography images of the control and the infarcted liver tissue obtained at 30 min p.i. shows a "dotted" distribution of MIH in accordance with the typical lobular arrangement of the intact liver tissue. At 30 min p.i. the uptake in the necrotic liver tissue is clearly lower than in the intact liver tissue (Fig. 3), both images have the same scale), but some parts of the infarcted lobe still show the same dotted appearance as the intact tissue. Close examination of the corresponding TTC staining of the infarcted tissue obtained at 30 min p.i. shows that the zone with high uptake on autoradiography is dark red after TTC staining whereas zones with low activity on autoradiography are pink after TTC staining. This indicates that the infarcted lobe still contains viable tissue (confirmed by TTC staining) which still actively extracts MIH from blood besides infarcted tissue in which relatively little MIH is retained.

At 24 h post injection the distribution pattern on autoradiography is the inverse of the image obtained at 30 min. The necrotic tissue now shows a higher concentration of ¹²³I distributed in a patchy fashion, whereas the intact liver tissue from which the MIH has cleared to the bile is now devoid of radioactivity.

Again in some animals the autoradiography showed a heterogenous activity distribution with low concentration in zones which after TTC staining are either red (viable tissue) or white (tissue lacking reperfusion) whereas zones which are pink on TTC staining are characterized by a relative high concentration of radioactivity. Comparison of the autoradiography image of the necrotic tissue obtained at 24 h p.i. with the image obtained after haematoxylin-eosin staining of the same tissue slice, shows that areas with high concentration of radioactivity are pink-purple coloured whereas areas with low radioactivity concentration either are pink (tissue lacking reperfusion) or purple (viable tissue coloured after H&E staining confirming that uptake of MIH is confined to reperfused areas which are necrotic.
The rat model of liver necrosis is relatively easy to set up yet it is not ideal for the evaluation of MIH as this tracer is cleared from the plasma by hepatobiliary excretion which complicates interpretation of the MIH liver images.
Therefore we have done an additional experiment using a rabbit model of myocardial infarction which is instrumentally more challenging but provides cleaner images as MIH does not appear to accumulate in the normal myocardium in neither mice or rats.
SPECT imaging after injection of MIH in the rabbit model shows a hot spot in the heart region, both at 24 h and 48 h post injection (Fig. 5 and 6).
The rabbit heart was excised and stained with TTC allowing to discernate the normal (deep red) from the infarcted tissue (pale pink).
Quantification of the concentation of MIH obtained by tissue counting shows a 7 fold higher uptake in the infarcted region (0.36 % of ID/g) compared to the normal region (0.05% of ID/g). The ratio is higher than the ratio observed for the rat liver model as in contrast to intact liver, the intact myocardium does not accumulate MIH and because a later timepoint (48h vs 24 h) was used in the rabbit experiment. The myocardial distribution of MIH was visualized with autoradiography and, in accordance with the rat study, a nice correlation was found between the areas not stained by TTC and the areas with higher uptake of radioactivity (Fig. 6). The autoradiography image shows that most of the MIH is found subendocardially suggesting that an occlusive myocardial infarct was induced in the rabbit so that delivery of MIH was restricted to diffusion from the ventricle attaining only the lateral border zone of the subendocardium. Quantification of the autoradiography image shows an uptake ratio of about 6 (comparable with the ratio observed after gamma counting) for the overall infarcted myocardium vs. the intact myocardium. When the region of the infarcted myocardium is limited to the subendocardial area, an uptake ratio as high as 16 is found for the infarcted tissue vs. the control tissue.
Regarding the mechanisms behind the observed necrosis-avidity of MIH it may be hypothetised that the mechanism is similar to the retention of hypericine in tumoral tissue. It is known that both hypericine and MIH bind extensively to HDL and LDL *in vivo* (37). In tumoral tissue it was found that the hypericine-lipoproteine complex extravasates in the tumor as a result of the higher permeability of the tumor vessels. Hypericine then leaves the lipoprotein and binds to the either collagen or cellular membranes.
Also in reperfused infarcted myocardium elevated permeability of the microvasculature is observed (42), so that MIH may use the same retention mechanism as hypericine in tumoral tissue. In detail studies are however required to confirm this hypothesis.
The present preliminary study has shown that MIH has necrosis avid properties and merits further evaluation as a potential useful tracer agent for tissue viability assessment. It is clear, however, that more investigations are needed, especially with respect to the dynamics of uptake, the earliest time-point when necrosis becomes visible and the mechanism of uptake in comparison with earlier reported necrosis avid tracer agents such as Tc-pyrophosphate and Tc-glucarate. In addition, further chemical optimization of this tracer appears to be indicated. The thyroid uptake observed after injection of MIH in both the rat and the rabbit suggests *in vivo* deiodination. For this reason and in view of the optimal characteristics of technetium-99m, a labeling with ^{99m}Tc would be more appropriate and will be included in further studies where MIH will serve as a lead compound.

### References

1. Flotats A, Carrió I. Non-invasive in vivo imaging of myocardial apoptosis and necrosis. Eur J Nucl Med Mol Imaging. 2003;30:615-630.
2. Buja LM, Tofe AJ, Kulkarni PV et al. Sites and mechanisms of localization of technetium-99m phosphorous radiopharmaceuticals in acute myocardial infarcts and other tissues. J Clin Invest. 1977; 60:724-740.
3. Khaw BA, Gold HK, Fallon JT, et al. Scintigraphic quantification of myocardial necrosis in patients after intravenous injection of cardiac myosin specific antibody. Circulation. 1986; 74:501-508.
4. Khaw BA, Yasuda T, Gold HK, et al. Acute myocardial infarction imaging with indium-111 labeled monoclonal antimyosin Fab fragments. J Nucl Med. 1987; 28:1671-1678.
5. Khaw BA, Fallon JT, Beller GA, Haber E. Specificity of localization of myosin-specific antibody fragments in experimental myocardial infarction: histologic, histochemical, autoradiographic and scintigraphic studies. Circulation. 1979;60:1527-1531.
6. Khaw BA, Scott J, Fallon JT, et al. Myocardial injury: quantitation by cell sorting initiated with antimyosin fluorescent spheres. Science. 1982;217:1050-1053.
7. Khaw BA. The current role of infarct avid imaging. Semin Nucl Med. 1999;29:259-270.
8. Frist W, Yasuda T, Segall G, et al. Noninvasive detection of human cardiac transplant rejection with indium-111 anti-myosin (Fab) imaging. Circulation. 1987;76:81-85.
9. Ballester M, Obrador D, Carrio I, et al. 111In-monoclonal antimyosin antibody studies after the first year of heart transplantation: identification of risk groups for developing rejection during long-term follow-up and clinical implications. Circulation. 1990;82:2100-2108.
10. Valdes Olmos RA, Carrio I, Hoefnagel M, et al. High sensitivity of radiolabelled antimyosin scintigraphy in assessing anthracycline related early myocyte damage preceding cardiac dysfunction. Nucl Med Commun. 2002;23:871-877.
11. Dec GW, Palacios IF, Yasuda T, et al. Antimyosin antibody cardiac imaging: its role in the diagnosis of myocarditis. J Am Coll Cardiol. 1990;6:97-104.
12. Narula J, Khaw BA, Dec GW, et al. Recognition of acute myocarditis masquerading as acute myocardial infarction. N Engl J Med. 1992;328:100-104.
13. Obrador D, Ballester M, Carrio I, et al. Active myocardial damage without attending inflammatory response in idiopathic dilated cardiomyopathy. J Am Coll Cardiol. 1993;21:1667-1671.
14. Obrador D, Ballester M, Carrio I, et al. Presence, evolving changes, and prognostic implications of myocardial damage detected in idiopathic and alcoholic dilated cardiomyopathy by 111In monoclonal antimyosin antibodies. Circulation. 1994;89:2054-2061.
15. Narula J, Petrov A, Pak KY, et al. Very early noninvasive detection of acute experimental nonreperfused myocardial infarction with 99mTc-labeled glucarate. Circulation. 1995;95:1577-1584.
16. Okada DR, Johnson G, Liu Z, et al. Early detection of infarct in reperfused canine myocardium using 99mTc-glucarate. J Nucl Med. 2004;45: 655-664.
17. Khaw BA, Nakazama A, O'Donell SM, et al. Avidity of technetium-99m glucarate for the necrotic myocardium: in vivo and in vitro assessment. J Nucl Cardiol. 1997;4:283.290.
18. Nelson J, Schmiedl U, Shankland E. Metalloporphyrins as tumor-seeking MRI contrast media and as potential selective treatment sensitizers. Invest Radiol. 1990;25: S71-S73.
19. Ni Y, Marchal G, Yu J, et al. Localization of metalloporphyrin induced "specific" enhancement in experimental liver tumors: a comparison between MRI, microangiographic and histologic findings. Acad Radiol. 1995;2:687-699.
20. Ni Y, Petré C, Miao Y, et al. Magnetic resonance imaging-histomorphologic correlation studies on paramagnetic metalloporphyrins in rat models of necrosis. Invest Radiol. 1997;32:770-779.
21. Maurer J, Strauss A, Ebert W, Bauer H, Felix R. Contrast-enhanced high resolution magnetic resonance imaging of pigmented malignant melanoma using Mn-TPPS4 and Gd-DTPA: experimental results. Melanoma Res. 2000;10: 40-46.
22. Marchal G, Ni Y. Use of porphyrin-complex or expanded porphyrin-complex as an infarction localization diagnosticum. U.S. patent No. 6,013,241. Priority date: May 11, 1994; Date of Patent: January 11, 2000.
23. Marchal G, Ni Y, Herijgers P, et al. Paramagnetic metalloporphyrins: infarct avid contrast agents for diagnosis of acute myocardial infarction by magnetic resonance imaging. Eur Radiol. 1996;6:2-8.
24. Ni Y, Marchal G, Herijgers P, et al. Paramagnetic metalloporphyrins: from enhancers for malignant tumors to markers of myocardial infarcts. Acad Radiol. 1996;3:S395-S397.
25. Herijgers P, Laycock SK, Ni Y, et al. Localization and determination of infarct size by Gd-mesoporphyrin enhanced MRI in dogs. Int J Cardiac Imaging. 1997;13:499-507.
26. Ni Y, Pislaru C, Bosmans H, et al. Validation of intracoronary delivery of metalloporphyrin as an in vivo "histochemical staining" for myocardial infarction with MR imaging. Acad Radiol. 1998;5:S37-S41.
27. Stillman AE, Wilke N, Jerosch-Herold M. Myocardial viability. Radiol Clin North Am. 1999;37:361.
28. Pislaru S, Ni Y, Pislaru C, et al. Noninvasive measurements of infarct size after thrombolysis with a necrosis-avid MRI contrast agent. Circulation. 1999,99:690-696.
29. Saeed M, Bremerich J, Wendland MF, et al. Reperfused myocardial infarction as seen with use of necrosis-specific versus standard extracellular MR contrast media in rats. Radiology. 1999;213:247-257.
30. Lim TH, Choi SI. MRI of myocardial infarction. J Magn Reson Imaging.1999;10:686-693.
31. Wendland MF, Saeed M, Lund G, Higgins CB. Contrast-enhanced MRI for quantification of myocardial viability. J Magn Reson Imaging.1999;10:694-702.
32. Choi SI, Choi SH, Kim ST, et al. Irreversibly damaged myocardium at MR imaging with a necrotic tissue-specific contrast agent in a cat model. Radiology. 2000;215:863-868.
33. Lee SS, Goo HW, Park SB, et al. MR imaging of reperfused myocardial infarction: comparison of necrosis-specific and intravascular contrast agents in a cat model. Radiology. 2003;226:739-747.
34. Ni Y, Miao Y, Bosmans H, et al. Evaluation of interventional liver tumor ablation with Gd-mesoporphyrin enhanced magnetic resonance imaging. Radiology. 1997;205:P319.
35. Pass HI. Photodynamic therapy in oncology: mechanisms and clinical use. J Natl Cancer Inst. 1993;85:443-456.
36. Chen B, Zupko I, De Witte PA. Photodynamic therapy with hypericin in a mouse P388 tumor model: vascular effects determine the efficacy. Int J Oncol. 2001;18:737-742.
37. Bormans,G.; Huyghe,D.; Christiaen,A. et al. Preparation, analysis and biodistribution in mice of iodine-123 labelled derivatives of hypericin. J Labelled Comp Radiopharm. 2004;47: 191-198.
38. Ni Y, Adzamli K, Miao Y, et al. MRI Contrast enhancement of necrosis by MP-2269 and Gadophrin-2 in a rat model of liver. infarction. Invest Radiol. 2001 ;36 97-103.
39. Ni Y, Dymarkowski S, Chen F, Bogaert J, Marchal G. Occlusive myocardial infarction: enhanced or not enhanced with necrosis avid contrast agents at magnetic resonance imaging. Radiology. 2002;225:603-605.
40. Rude R, Parkey RW, Bonte FJ, et al. Clinical implications of the "doughnut" pattern of uptake in myocardial imaging with technetium-99m stannous pyrophosphate. Circulation. 1977;56:146-147.
41. Lavie G, Mazur Y, Lavie D, Meruelo D. The chemical and biological properties of hypericin: a compound with a broad spectrum of biological activities. Med Res Rev 1995;15:111-119.
42. Bremerich J, Wendland MF, Arheden H, et al. Microvascular injury in reperfused infarcted myocardium: noninvasive assessment with contrast-enhanced echoplanar magnetic resonance imaging. J Am Coll Cardiol 1998;32:787-793.
43. Huyghe, D., Bormans, G.M., Verbeke, K.A., Verbruggen, A.M., de Witte, P.A. Preparation of mono-[123I]-Iodohypericim, biodistribution in a mouse RIF-1 tumour model and protein binding in human plasma. Journal of nuclear medicine, vol. 44, no. 5 Suppl, May 2003, page 303P, 50th Annual Meeting of the Society of Nuclear Medicine; New Orleans, LA, USA ; June 21-25, 2003 Abstract No.1086

### Tables

**Table 1. Biodistribution of MIH in rats expressed as % of injected dose per gram tissue (meant±SD, n=3)**

| | Time p.i. | |
|---|---|---|
| | 30 min | 24h |
| Blood | 1.34±0.38 | 0.10±0.01 |
| Stomach | 0.03±0.00 | 0.37±0.13 |
| Small intestine | 0.09±0.03 | 0.27±0.02 |
| Large intestine | 0.03±0.01 | 1.47±0.13 |
| Kidneys | 0.35±0.05 | 0.18±0.02 |
| Spleen | 2.19±0.18 | 0.43±0.07 |
| Normal liver | 3.80±1.39 | 0.55±0.15 |
| Necrotic liver | 1.91±0.46 | 1.71±0.32 |
| Heart | 0.47±0.13 | 0.24±0.08 |
| Lungs | 1.60±0.58 | 1.06±0.30 |
| Muscle | 0.03±0.01 | 0.03±0.00 |
| Thyroid | 0.16±0.02 | 9.25±3.25 |
| | | |
| Necrotic liver/Intact liver | 0.57±0.28 | 3.18±0.49 |
| Necrotic liver/Blood | 1.44±0.10 | 17.40±1.19 |
| Necrotic liver/Muscle | 75.80±16.34 | 57.09±4.24 |

## Claims

1. Use of a compound comprising phenanthro [1,10,9,8-opqra]perylene-7,14-dione for the manufacture of an imaging agent for obtaining an image of ischemic, infarcted or necrotic tissue.

2. The use of claim 1 wherein said compound has photosensitising activity.

3. The use of claim 1 or claim 2 wherein said compound is hypericin, pseudohypericin or a derivative thereof.

4. The use of claim 1 or claim 2 wherein said compound is stentorin or a derivative thereof.

5. The use of claim 1 or claim 2 wherein said compound is a fringelite or a derivative thereof.

6. The use of claim 1 or claim 2 wherein said compound is a gymnochrome or a derivative thereof.

7. The use of claim 1 or claim 2 wherein said compound is blepharismin or a derivative thereof.

8. The use of any of claims 1 to 7 wherein said compound is conjugated to a radionuclide.

9. The use of claim 8 wherein said compound is hypericin.

10. The use of any of claims 1 to 7 wherein said compound is conjugated to a radiopaque material.

11. The use of any of claims 1 to 7 wherein said compound is conjugated to a material that enhances the effects of magnetic resonance imaging.

12. A pharmaceutical composition comprising a hypericin or a derivative thereof being conjugated to a radiopaque material or a radionucleide, wherein said radionucleide is selected from the group consisting of Tc-99m, 1-125, I-111, In-113m and G-67.

13. A pharmaceutical composition comprising a phenanthro[1,10,9,8-opqra]perylene-7,14-dione compound conjugated to a radionucleide or a radiopaque material wherein said phenanthro[1,10,9,8-opqra]perylene-7,14-dione compound is selected from pseudohypericin, stentorin, the fringelites, the gymnochromes, and blepharismin.

## Patentansprüche

1. Verwendung einer Verbindung, umfassend Phenanthro[1,10,9,8-opqra]perylen-7,14-dion zur Herstellung eines bildgebenden Mittels, um eine Abbildung eines Gewebes mit einer Ischämie, einem Infarkt oder einer Nekrose zu erhalten.

2. Verwendung nach Anspruch 1, wobei die Verbindung eine photosensibilisierende Aktivität besitzt.

3. Verwendung nach Anspruch 1 oder Anspruch 2, wobei die Verbindung Hypericin, Pseudohypericin oder ein Derivat hiervon ist.

4. Verwendung nach Anspruch 1 oder Anspruch 2, wobei die Verbindung Stentorin oder ein Derivat hiervon ist.

5. Verwendung nach Anspruch 1 oder Anspruch 2, wobei die Verbindung ein Fringelit oder ein Derivat hiervon ist.

6. Verwendung nach Anspruch 1 oder Anspruch 2, wobei die Verbindung ein Gymnochrom oder ein Derivat hiervon ist.

7. Verwendung nach Anspruch 1 oder Anspruch 2, wobei die Verbindung Blepharismin oder ein Derivat hiervon ist.

8. Verwendung nach einem der Ansprüche 1 bis 7, wobei die Verbindung an ein Radionuklid konjugiert ist.

9. Verwendung nach Anspruch 8, wobei die Verbindung Hypericin ist.

10. Verwendung nach einem der Ansprüche 1 bis 7, wobei die Verbindung an ein radiopaques Material konjugiert ist.

11. Verwendung nach einem der Ansprüche 1 bis 7, wobei die Verbindung an ein Material konjugiert ist, welches die Wirkungen der magnetischen Resonanzbildgebung verstärkt.

12. Pharmazeutische Zusammensetzung, umfassend ein Hypericin oder ein Derivat hiervon, welches an ein radiopaques Material oder ein Radionukleid konjugiert ist, wobei das Radionukleid ausgewählt ist aus der Gruppe, bestehend aus Tc-99m, I-125, I-111, In-113m und G-67.

13. Pharmazeutische Zusammensetzung, umfassend eine Phenanthro[1,10,9,8-opqra]perylen-7,14-dion-Verbindung, die an ein Radionukleid oder radiopaques Material konjugiert ist, wobei die Phenanthro[1,10,9,8-opqra]perylen-7,14-dion-Verbindung ausgewählt ist aus Pseudohypericin, Stentorin, den Fringeliten, den Gymnochromen und Blepharismin.

## Revendications

1. Utilisation d'un composé comprenant une phénanthro[1,10,9,8-opqra]pérylène-7,14-dione destiné à la fabrication d'un agent d'imagerie pour obtenir une image d'un tissu ischémique, infarci ou nécrosé.

2. Utilisation selon la revendication 1, d ans laquelle ledit composé a une activité de photosensibilisation.

3. Utilisation selon la revendication 1 ou la revendication 2, dans laquelle ledit composé est l'hypéricine, la pseudohypéricine ou un dérivé de celle-ci.

4. Utilisation selon la revendication 1 ou la revendication 2, dans laquelle ledit composé est la stentorine ou un dérivé de celle-ci.

5. Utilisation selon la revendication 1 ou la revendication 2, dans laquelle ledit composé est une fringélite ou un dérivé de celle-ci.

6. Utilisation selon la revendication 1 ou la revendication 2, dans laquelle ledit composé est un gymnochrome ou un dérivé de celui-ci.

7. Utilisation selon la revendication 1 ou la revendication 2, dans laquelle ledit composé est la blépharismine ou un dérivé de celle-ci.

8. Utilisation selon l'une quelconque des revendications 1 à 7, dans laquelle ledit composé est conjugué à un radionucléide.

9. Utilisation selon la revendication 8, dans laquelle ledit composé est l'hypéricine.

10. Utilisation selon l'une quelconque des revendications 1 à 7, dans laquelle ledit composé est conjugué à un matériau radio-opaque.

11. Utilisation selon l'une quelconque des revendications 1 à 7, dans laquelle ledit composé est conjugué à un matériau qui renforce les effets de l'imagerie par résonance magnétique.

12. Composition pharmaceutique comprenant une hypéricine ou un dérivé de celle-ci qui est conjugué(e) à un matériau radio-opaque ou à un radionucléide, dans laquelle ledit radionucléide est choisi dans le groupe constitué par Tc-99m, I-125, I-111, In-113m et G-67.

13. Composition pharmaceutique comprenant un composé phénanthro[1,10,9,8-opqra]pérylène-7,14-dione conjugué à un radionucléide ou à un matériau radio-opaque, dans laquelle ledit composé phénanthro[1,10,9,8-opqra]pérylène-7,14-dione est choisi parmi la pseudohypéricine, la stentorine, les fringelites, les gymnochromes et la blépharismine.
